Europäisches Patentamt

European Patent Office

Office européen des brevets

(11) Publication number: **0 129 483**

**A1**

(12) **EUROPEAN PATENT APPLICATION**

(21) Application number: 84401241.9

(22) Date of filing: 15.06.84

(51) Int. Cl.³: **A 61 N 1/36**
**A 61 B 5/04**

(30) Priority: 15.06.83 US 504700

(43) Date of publication of application:
27.12.84 Bulletin 84/52

(84) Designated Contracting States:
DE FR GB NL

(71) Applicant: MEDTRONIC, INC.
3055 Old Highway Eight
Minneapolis Minnesota 55440(US)

(72) Inventor: Shturman, Leonid, M.D.
100 West Avenue/613 West
Jenkintown Pennsylvania 19046(US)

(72) Inventor: Badzinski, John D.
11103 Foley Blvd.
Coon Rapids, MN 55433(US)

(72) Inventor: McNichols, Larry A.
11204 Arrowhead NW
Coon Rapids, MN 55433(US)

(74) Representative: Bressand, Georges et al,
c/o CABINET LAVOIX 2 Place d'Estienne d'Orves
F-75441 Paris Cedex 09(FR)

(54) Organ capture detection for electrical stimulators.

(57) A medical stimulator is disclosed in which a number of sensing electrodes are placed along the conductive path of an organ, for example, along the jejunum at a location remote from the natural pacemaker in the proximal duodenum. The pacing electrodes are positioned adjacent the group of sensing electrodes and pacing pulses of a frequency higher than the frequency of a natural pacemaker of the organ are applied to the pacing site(s). In order to be effective, however, the physician using the apparatus must know that capture of the organ has been obtained by the pacing pulses. The present invention achieves this result by evaluating the sensed pulse signals that are received at each of the sensing electrodes in a timed sequence to determine if pacing of the organ is under the control of the applied pacing pulses.

Fig. 3

EP 0 129 483 A1

ORGAN CAPTURE DETECTION FOR
ELECTRICAL STIMULATORS

## BACKGROUND OF THE INVENTION

The present invention relates in general to electrical stimulation of bodily organs and, in particular, to stimulation of the gastro-intestinal tract to treat such disorders as post-gastrectomy dumping syndrome, short-bowel syndrome, malabsorption and other disorders. Medical and surgical therapy has been attempted for these post-operative syndromes, but these generally have produced variable results with few instances of restoration of perfect health.

The present invention is especially adapted for electrical stimulation of organs which have their own natural pacemaker. In the case of the gut, for example, this system serves to overcome the too-rapid emptying of the stomach, or too-short a transit time of the bowel. The gastro-intestinal tract moves nutrient and waste material through a process called peristalsis, which involves the contraction of muscles in response to natural electrical stimuli. In order to treat gastro-intestinal disorders with artificial stimulation, it is necessary to supply the stimulation pulses at a higher frequency than the natural peristallic action so as to pace the gut in a retrograde manner, thereby extending emptying times so as to overcome disorders related to the too-rapid emptying of the stomach or short transit time of the bowel.

The effective utilization of these artificial stimulation pulses requires a precise way of determining when the artificial stimulation pulses obtain control, or capture, of the bowel instead of the natural ones from the organs' pacemaker. The present invention relates to a precise determination of the capture of the artificial stimulation pulses and provides a positive identification of capture of the organ.

-2-

It has been suggested that the rhythmic electrical activity of the small intestine may be described as a system of loosely-coupled oscillators. It has also been proposed that a natural pacemaker is located in an undefined area of the duodenum, which controls this rhythmic activity. Such a proposal is described in the article entitled, "Simulation Of The Electrical And Mechanical Gradient Of The Small Intestine", by Thomas S. Nelson and John C. Becker, which appeared in the American Journal of Physiology, Volume 214, No. 4, April, 1968, pages 749-757. Related articles include the one entitled, "Control Of Intestinal Rhythmic Contractions By A Duodenal Pacemaker", authored by Robert Hasselbrack and J. E. Thomas in the American Journal of Physiology, Vol. 201, 1961, pages 955-960.

The existence of multiple pacemakers along the bowel is hypothesized in the article, "Nature Of The Intestinal Slow-wave Frequency Gradient", by Nicholas Diamant and Alex Bortoff in the American Journal of Physiology, Volume 216, No. 2, February, 1969, Pages 301-307. The Diamant article also discusses the detection of slow-waves through the jejunum of a cat, which were recorded with a number of closely spaced suction electrodes. Another related article is entitled, "The Controls of Gastro-intestinal Movements: Some Old And New Views", by James Christensen, which appeared in the New England Journal of Medicine, Volume 285, No. 2, Pages 85-98, 1971.

The placement of pacing electrodes in the stomach to change the normal emptying rate of the stomach under the control of artificial stimulating pulses, has been described in the article entitled, "Role Of The Gastric Pacesetter Potential Defined By Electrical Pacing", in the Canadian Journal of Physiology and Pharmacology, Volume 50, 1972, Pages 1017-1019 by Keith Kelly and Richard LaFores. Keith Kelly also described a similar arrangement in "Motility Of The Stomach And Gastro-Duodenal Junction",

-3-

in the book entitled <u>Physiology Of The Gastro-intestinal Tract</u>, Rayburn Press, New York, 1981.

## BRIEF DESCRIPTION OF THE DRAWINGS

The present invention is disclosed by reference to the drawings in which:

FIG. 1 is a diagramatic showing of a human stomach and a portion of the gastro-intestinal tract which shows pacing and sensing sites that may be employed in conjunction with the present invention;

FIG. 2 is a waveform representation which shows waveforms that are developed in the present invention;

FIG. 3 is a block diagram of a telemetry transmitter employed in the present invention; and,

FIG. 4 is a block diagram of the receiver and control portion of the invention.

## TECHNICAL DESCRIPTION OF THE INVENTION

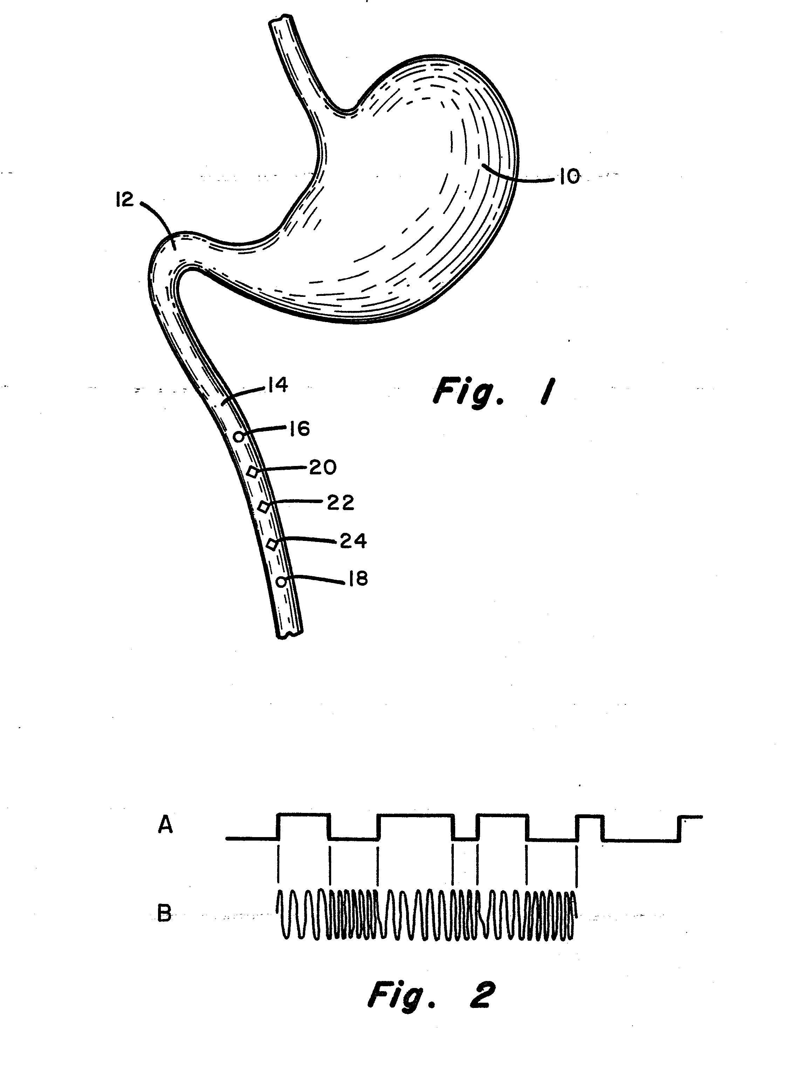

FIG. 1 shows a diagramatic view of the human stomach 10, the duodenum 12 and the jejunum 14. As described above, it is possible to postulate the existence of a electrical pacemaker in the general area of the duodenum, at which slow electrical waves of activity originate and progress down the jejunum. It has been further postulated that there are a multiple of possible pacemaker sites which decrease in frequency along the jejunum as it moves away from the duodenum. For example, as shown in FIG. 1, if site 16 acted as a pacemaker site, it would have a lower natural frequency than the normally controlling pacemaker site in the duodenum 12. Correspondingly, the even more remote site 18 would be at a lower natural frequency than the site 16.

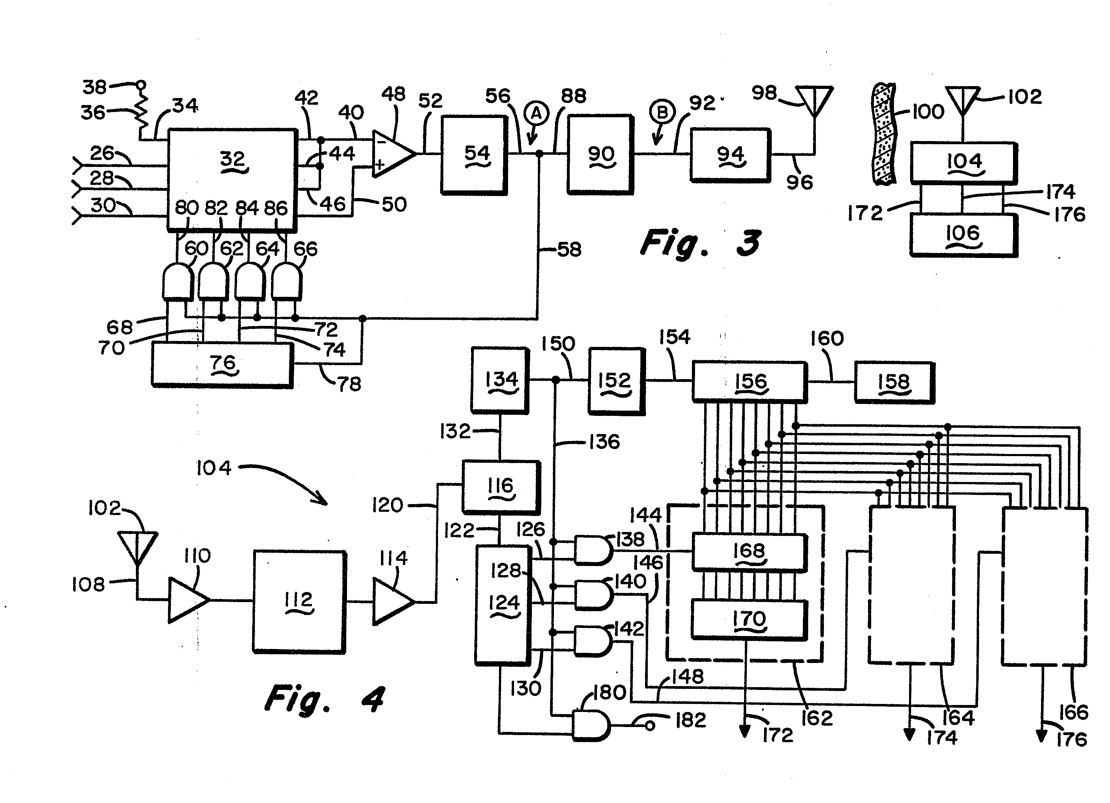

The approximately even spaced sites 20, 22 and 24 of the present invention are locations where sensing electrodes may be placed on the jejunum, either externally or internally. The site 18, in the present invention, is a location where preferably a bi-polar pair of pacing electrodes are attached. Possibly, a unipolar lead may be employed at the site 18 in combination with an indifferent lead. The pacing electrodes at the site 18 are connected to a conventional gastro-intestinal pacer, (not shown), while the sensing electrodes at the sites 20, 22 and 24 are coupled to the leads 26, 28 and 30 of FIG. 3. The leads 26, 28 and 30 are in turn, coupled to the multiplexer 32. A fourth input to the multiplexer is supplied on the lead 34 through the resistor 36 from the B+ power supply, which is coupled to the terminal 38.

The input signals received on the leads 26, 28 and 30 are multiplexed out on the single output line 40, which is connected to the output lines 42, 44 and 46, which respectively correspond to the input leads 26, 28 and 30. The output line 40 of the multiplexer 32 is connected to the inverting input terminal of a differential amplifier 48. Since the natural electrical stimuli involved in the peristaltic process may be represented as a series of natural pacing sites, which decrease in frequency along a path that extends downward along the jejenum, control is normally assumed by the highest frequency pacing site. That is located in the proximal duodenum. It has been verified that a natural electrical stimulation signal produced by a natural pacemaker will result in a timed sequence of signals being sensed in succession at a series of electrodes, such as those at the sensing sites 20, 22 and 24.

In the furtherance of the present invention, the pacing electrodes are positioned at a site 18 below the series of sensing sites 20, 22 and 24 in a manner such that pulses applied to the pacing electrodes will assume control, or "capture" of pacing of the organ. In this manner, waves generated at the pacing site can travel in a retrograde manner up the jejunum, thereby controlling the post-gastrectomy dumping syndrome. When bi-polar electrodes are used for pacing at the site 18, they will be generally spaced on the order of 5-8 millimeters apart.

Pulse width may be on the order of 40 milliseconds and pulse amplitude may be approximately 8 volts.

In order to insure positive capture by the artificial pacemaker and to minimize the energy required by pacing, it is necessary to positively know when capture occurs. Pulse width and amplitude generally may be set wide and large enough to insure initial capture and they may then be decreased until capture is lost. Then they are increased to a safety margin level above the minimum capture level, so as to insure capture while minimizing energy requirements. The system described herein allows such precise determination to be made of when capture occurs.

When pacing is taking place at the site 18, retrograde waves will travel toward the duodenum past the sensing sites 24, 22 and 20 in that order and, therefore, the leads 26, 28 and 30 will receive signals in the same sequence. Signals on the leads 26, 28 and 30 are coupled to the multiplexer 32, which although A.C. signals share a relatively slow change rate that allows for these signals to be treated as D.C. levels during sensing.

The output line 50 from the multiplexer 32 is coupled to the non-inverting input terminal of the amplifier 48. This line is multiplexed to receive a signal from the input lead 34, which monitors the power supply. The signals on the lines 40 and 50 occur at different times and thus, the differential amplifier 48 is not employed in a differential mode, but is used instead to meet the different amplification requirements of the sensed wave signals provided on the line 40 and the battery level signal provided on the line 50.

The output of the amplifier 48 is supplied on its output line 52 to an amplitude-to-pulse width converter 54 which is triggered by the input signals it receives, and it provides a waveform, such as the waveform A of FIG. 2, which consists of a train of variable width pulses that correspond to the input signals that are sensed on the lines 26, 28 and 30. For example, the left-most pulse in FIG. 2 may represent the last signal received on the line 30, the next left-most pulse is the signal received on the line 28 and the third pulse to the right is the last signal received on the line 26. An amplitude-to-pulse width converter may be constructed with a converter connected in this manner. By utilizing a ICM 7555 low power general purpose timer circuit manufactured by Intersil. Specifically the converter 54 may be implemented with a 7555 timer if line 52 of FIG. 3 is connected to pin 8 of the 7555; line 56 is connected to pin 3; pins 2 and 6 are coupled to a capacitor, on the order of 0.01 microfarads, the other end of which is coupled to ground; a resistor, on the order of 4.7 kilohms is coupled between pins 6 and 7; another resistor on the order of 47 kilohms is coupled from pin 7 to a B+ power supply; pin 2 is grounded, and B+ is also coupled to pins 4 and 8. These connections correspond to astable operation of the 7555 circuit with control pin 5 being used to modulate the frequency of oscillator of the circuit, but where the circuit oscillates at such a low rate that pulse width modulation rather than frequency modulation results. The "low" portion of each pulse of the output pulse is approximately of the same width, while the "high" portion of each pulse varies in accordance with the amplitude of the input pulse that is being applied to the circuit at the time. (The terms "low" and "high" as used herein merely refer to digital logic levels, and hence they obviously may be interchanged).

The output waveform A, which appears on the output line 56 of the multi-vibrator 54 is supplied on the interconnecting line 58 as a gating signal to the AND gates 60, 62, 64 and 66. The other input lines 68, 70, 72 and 74, respectively, are supplied by a recirculating counter 76. The signal which appears on the line 58 is also supplied on the line 78 as a counting signal to the counter 76. Each time that one of the pulses of waveform A changes from a "high" to a "low" state on the line, the counter 76 increments its count. This allows gates 60, 62, 64 and 66 to supply an output signal on a corresponding one of the lines 80, 82, 84 and 86 in sequence so that each gate is selected at a time in a predetermined cyclic order. For example, if the left-most pulse represented a sensed signal on the line 26, the termination of this signal will allow the counter to activate gate 62 and deactivate gate 60. Thus, the next signal that appears on the line 28 will be coupled to the multiplexer 32. The gates 60, 62 and 64, respectively corresponded to sensing lines 26, 28 and 30, while the gate 66 is related to the battery sense input line 34. The counter 76 is reset following the application of four counting pulses so that control is resumed by gate 60 following termination of control by gate 66.

The waveform A of FIG. 2, which is supplied on the line 56, is also supplied on the line 88 to a voltage-to-frequency converter 90. The voltage-to-frequency converter 90 takes the waveform A from the amplitude-to-pulse width converter 54 and converts it to waveform B of FIG. 2. The converter 54 is an oscillator circuit which is frequency modulated (F.M.) by the "high" level of the output pulse to provide a F.M. output signal, the center frequency of which may be on the order of 175 kHz.

Waveform B is supplied as a signal on the output line 92 to the RF output circuit 94. The RF output circuit 94 is coupled to the antenna 98, which transmits the RF modulation output through the skin 100 of a patient, as seen in FIG. 2. When waveform A is at its "high" level, a relatively low frequency is transmitted by the antenna 98; and when the waveform A is at its "low" level, a relatively high frequency will be transmitted as shown by waveform B. In this manner, the signals sensed on the lines 26, 28, 30 and 34 are converted into frequency-modulated signals which are transmitted from a device that may be implanted in the patient to an external receiver.

The antenna 102 is a receiving antenna of the receiver and is coupled to the control section 104 of the receiver, which in turn, is coupled to a microprocessor 106 which may be utilized to provide analysis of the received signals. The control section 104 of the receiving unit of the present invention is shown in more detail in the block diagram of FIG. 4.

The pulse width of waveform A is directly related to the amplitude of the signals on the sense lines coupled to the multiplexer 32. Thus, by measuring the relative widths of the pulses of waveform A and the rate of change of these widths, the microprocessor can detect the direction of passage of a wave past the sensing sites 20, 22 and 24. Measurement of pulse width is conveniently accomplished by counting the number of pulses that a "high" frequency clock develops while the pulse is at a "high" level, and the rate of change is provided by recording the change of this count over a period of time. Other detection schemes may alternately be employed for the purpose. For example, detection of the peak amplitude of the signals on each sensing electrode may be used to detect passage of the retrograde pacing wave and thus capture of the organ.

The signal received on the receiving antenna 102 is coupled at the line 108 to an input amplifier 110 which in turn, is coupled to a phase-lock loop demodulation circuit 112. The output of the demodulation circuit 112 is coupled through the amplifier 114 on the line 120 to a level-shifting circuit 116. The purpose of the level-shifting circuit 116 is to convert the signals that are supplied by the amplifier 114 to a logic level signal which is supplied on the line 122 to a recirculating counter 124 to increment the count of the counter upon the change of output of the circuit 116 from a "high" to a "low" level (or alternately from a "low" to a "high" level.

The counter 124 is used to supply gating signals on the lines 126, 128 and 130 to gate on the AND gates 138, 140 and 142, which correspond as far as information is concerned to the AND gates 60, 62 and 64, of the transmitter. The counter 124 may also be employed to gate on the AND gate 180 which corresponds to the AND gate 66 at the transmitter for sensing the battery power supply. The logic level signals provided by the level-shifting circuit 116 are also coupled on the line 132 to a monostable multi-vibrator 134 (one-shot). The multi-vibrator 134 is triggered by the signal on the line 132 so that it supplies a pulse on a line 136 sequentially to AND gates 138, 140, 142 and 180. This allows the counter 124 to select one of the gates 138, 140, 142 and 180 sequential manner, corresponding to the sequential selection of the gates 60, 62, 64 and 66 of the transmitter.

The output of the one-shot 134 is also supplied on the line 150 to a second monostable multi-vibrator 152 (one-shot). The output of the one-shot 152 is coupled on the line 154 to an eight-stage counter 156, which is driven by clock pulses from the clock 158 that are supplied on the line 160 to the counter. The output of the multi-vibrator 152 is employed to reset the counter 156 so that as it cycles through its counting stages it will be reset when the next gate of the gates 138, 140, 142 and 180 is selected. Thus, the one-shot 134 will sequentially provide the selecting signals on the lines 144, 146 or 148 so as to select one of the decoding circuits 162, 164 or 166 for actuation.

The decoding circuits 162, 164 and 166 each have a latching input stage 168 which latches in the count of the counter 156 that takes place during the time the enabling signal is supplied on the line 144. Once latched in the latches 168, the signal will be decoded by a digital-to-analog circuit 170 to provide an analog output signal on the corresponding output line 172, 174 or 176 which is an analog representation of the corresponding pulse width of the associated pulse of waveform A, and hence of the amplitude on the corresponding input line to the multiplexer 32 of FIG. 3. (The microprocessor 106 may have its own analog-to-digital converter incorporated into it so that it can receive the analog signals, convert them to digital form and process them further in a digital manner.)

In addition to the decoding circuits 162, 164 and 166 illustrated in FIG. 4, which provide analog output signals representative of signals on the sensing lines 26, 28 and 30, a similar circuit (not shown) may also be provided for developing an analog signal representative of the battery supply signal on the line 34. This circuitry would be selected by the AND gate 180 and its output signal on the line 182.

Although a particular embodiment of the present invention has been described, it will be recognized that there are obvious modifications within the scope of the present invention that are intended to be covered within the scope of the appended claims. For example, although the sensing electrodes have been shown as being arranged along a linear path, it is apparent to those skilled in the art that other patterns which provide the desired sequential sensing of the pacing pulses of the present invention are within the scope of the present invention. For example, concentric circular electrodes which surround a pacing site may be used. Other configurations and modifications of the present invention within the scope of the invention will also be apparent to those skilled in the art.

What is claimed is:

CLAIMS

1.    In a medical stimulator comprising pulse generator means for applying electrical stimulating pulses at a pacing site of an organ that normally has a natural pacemaker control site which initiates electrical pulses wherein said naturally produced pacemaker pulses travel in a predetermined direction along a conductive path in said organ, and sensing means for sensing the physiological electrical response of a subject to said stimulating pulses; the improvement wherein said sensing means comprises more than one adjacent detection means spaced along said path and located intermediate said natural pacemaker and said pacing site, said pulse generator means is adapted to supply said stimulating pulses at a pulse repetition rate that is detectably greater than the rate of the natural stimulating pulses generated by said natural pacemaker and detecting means are coupled to said sensing means for determining if pulses traveling in said organ are natural pulses that travel in said predetermined direction, or if they are stimulating pulses applied by said pulse generator means which travel in the opposite direction which signify capture of the organ by said pulse generator means.

2.    In a medical stimulator comprising pulse generator means for applying electrical stimulating pulses at a pacing site of an organ that normally has a natural pacemaker control site which initiates electrical pulses, wherein said naturally produced pacemaker pulses travel along a conductive path in said organ which comprises a chain of potential pacemaker sites, each of which have a decreasing natural stimulation frequency in accordance with their distance along said path from said pacemaker control site and detection means located at a plurality of said potential pacemaker sites, wherein the frequency of pulses supplied by said pulse generator means is higher than the natural frequency of said control site; the improvement comprising, sensing means coupled to said detection means for determining the response of said detection means to said stimulating pulses in said organ in order to indicate whether or not the pulse generator means has capture of said organ, wherein said pulse generator means is located in the vicinity of one of said lower frequency potential natural pacing sites and said sensing means is responsive to the detection pattern sensed from said detection means by said sensing means.

3. In a medical stimulator as claimed in claim 2 the improvement wherein said detection means are located intermediate said pacemaker control site and said pulse generator means.

4. A medical stimulator comprising pacing means, a plurality of spaced-apart detection means positioned in a conductive pattern relative to an electrically stimulatable organ of a subject at locations remote from said pacing means, pulse generator means coupled to said pacing means for supplying pacing pulses to said pacing means and monitor means coupled to said detection means constructed to provide an indication of electrical capture of said organ by said pulse generator means when pulse signals are detected in a sequential manner at each of said detection means.

5. A medical stimulator as claimed in claim 4 wherein said sequential manner begins with the detection means farthest from said pulse generator means.

6. A medical stimulator as claimed in claim 4 wherein said sensing means comprises transmitter means, capable of being implanted in said subject and said monitor means comprises an external receiver which receives signals transmitted remotely from said subject.

7. A medical stimulator as claimed in claim 6 wherein said transmitter means comprises a multiplexer coupled to each of said detecting means, gate means for sequentially controlling said multiplexer to provide an output signal on a single output line, wherein said gate means is constructed to allow said multiplexer to receive inputs from each of said detecting means in a sequential manner so that the next detecting means in the sequence is multiplexed out of the multiplexer only after the signal received on the preceding detection means of the sequence has terminated.

8. A medical stimulator as claimed in claim 7 comprising a battery power supply means coupled to said multiplexer wherein said multiplexer means provides an output indication of said battery supply in a sequential manner interspersed with the sequential signals from said detecting means.

9. A medical stimulator as claimed in claim 8 wherein said gate means comprises gate selection means. Timing means coupled to the output of said multiplexer means for timing the occurrence of the activation of said gate means and counter means coupled to receive said activation signal from said timing means so as to select one of said gate selecting means in a sequential manner.

10. A medical stimulator as claimed in claim 9 wherein said receiver means comprises frequency-modulated demodulation means for converting frequency-modulated signals into pulse-width-modulated signals, and signal means coupled to said demodulating means for providing output signals representative of the width of the signals detected by said detection means in a sequential manner.

11. A medical stimulator as claimed in claim 10 wherein said signal means comprises signal gate means, a first counting means coupled to said signal gate means to activate each of said signal gate selection means.

12. A medical stimulator as claimed in claim 11 comprising signal means comprising second counted means coupled to said gate activation means so as to be reset upon the activation of said gate activation means, clock means coupled to supply counting pulses to said second counting means and a plurality of digital-to-analog converting means each coupled to one of said signal gate means so as to be selectively actuated in sequence by said signal gate means for a period of time that corresponds to the width of the associated pulse of the pulse-width-
modulated signal.

13. A medical stimulator as claimed in claim 12 wherein said sequential manner begins with the detection means farthest from said pulse generator means.

Fig. 1

Fig. 2

Fig. 3

Fig. 4

0129483

2/2

**European Patent Office**

**EUROPEAN SEARCH REPORT**

Application number

## DOCUMENTS CONSIDERED TO BE RELEVANT

EP 84401241.9

| Category | Citation of document with indication, where appropriate, of relevant passages | Relevant to claim | CLASSIFICATION OF THE APPLICATION (Int. Cl. ³) |
|---|---|---|---|
| A,D | CANADIAN JOURNAL OF PHYSIOLOGY AND PHARMACOLOGY, vol. 50, 1972 <br> K. KELLY, R. LA FORCE "Role of the Gastric Pacesetter Potential Defined by Electrical Pacing" <br> pages 1017-1019 <br><br> * Page 1017, right column, last paragraph - page 1018, right column, second paragraph; fig. 1 * <br><br> -- | 1-4 | A 61 N 1/36 <br> A 61 B 5/04 |
| A | EP - A1 - 0 057 048 (CHATTANOOGA CORP.) <br><br> * Abstract; page 3, lines 31-36 * <br><br> -- | 1-4 | |
| A | CH - A5 - 586 047 (STRAUMANN) <br> * Claims; fig. 1,4 * <br><br> -- | 1,2,4 | **TECHNICAL FIELDS SEARCHED (Int. Cl. ³)** |
| A | DE - A - 1 919 526 (NOLTE) <br> * Page 1, line 14 - page 3, line 10; fig. * <br><br> -- | | A 61 N <br> A 61 B |
| A | DE - A1 - 3 130 104 (MESSERSCHMITT-BOLKOW-BLOHM) <br><br> * Abstract; claim 1; fig. 1 * <br><br> -- | 1-4,6 | |
| A | EP - A1 - 0 057 561 (BIO MEDICAL) <br> * Abstract; fig. 1 * <br><br> -- | 4,6,7 | |

The present search report has been drawn up for all claims

| Place of search | Date of completion of the search | Examiner |
|---|---|---|
| VIENNA | 10-09-1984 | NEGWER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82

**European Patent Office**

**EUROPEAN SEARCH REPORT**

## DOCUMENTS CONSIDERED TO BE RELEVANT

| Category | Citation of document with indication, where appropriate, of relevant passages | Relevant to claim |
|---|---|---|
| A | US - A - 4 345 603 (SCHULMAN) <br> * Abstract; column 1, lines 38-49 * <br> ---- | 4,6-8 |

**CLASSIFICATION OF THE APPLICATION (Int Cl )**

**TECHNICAL FIELDS SEARCHED (Int Cl )**

EPO Form 1503.2 06.78